# EUROPÄISCHE PATENTANMELDUNG

(11) **EP 1 013 259 A1**
(43) Veröffentlichungstag der Anmeldung: **28.06.2000**
(21) Anmeldenummer: 99120515.4
(22) Anmeldetag: 15.10.1999
(51) Int. Cl.: A61K 7/13

(54) **Mittel zum Färben von Fasern mit einem Gehalt an 1,3-Indandionen**

(30) Priorität: 15.12.1998 DE 19857848
(71) Anmelder: Wella Aktiengesellschaft, 64274 Darmstadt (DE)
(72) Erfinder: Göttel, Otto, Dr., 1723 Marly (CH); Mettler, Sandra, 1723 Marly (CH); Hayoz, André, 1724 Senèdes (CH); Pirrello, Aline, 1762 Givisiez (CH)

(57) **Zusammenfassung**

Die vorliegende Erfindung betrifft ein Mittel zum Färben von Fasern, insbesondere Keratinfasern, wie zum Beispiel menschlichen Haaren, welches dadurch gekennzeichnet ist, daß es mindestens ein 1,3-Indandion der allgemeinen Formel (I) enthält, 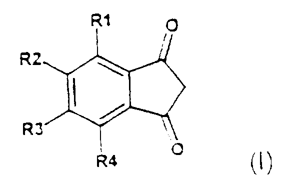 wobei die Reste R₁, R₂, R₃ und R₄ untereinander gleich oder verschieden sein können und die folgende Bedeutung haben: Wasserstoff, ein Halogenatom, eine Nitrogruppe, eine Aminogruppe, eine Acetylaminogruppe, eine Hydroxygruppe, eine Acetoxygruppe, eine Methylgruppe oder Ethylgruppe, und so weiter; sowie ein Verfahren zum Färben von Fasern.

## Beschreibung

Gegenstand der Erfindung sind Färbemittel mit einem Gehalt an 1,3-Indandionen.

Auf dem Haarfarbsektor besteht nach wie vor ein steigender Bedarf nach kastanienbraunen und kupferfarbigen bis roten Nuancen. Diese Nachfrage kann beispielsweise durch die Bereitstellung von Oxidationshaarfärbemitteln auf der Basis von bestimmten Entwicklersubstanzen, wie zum Beispiel p-Aminophenol-Derivaten, mit bestimmten Kupplersubstanzen, wie zum Beispiel m-Aminophenol- oder m-Phenylendiamin-Derivaten, befriedigt werden (permanente Färbung). Mit den genannten Oxidationsfärbemitteln lassen sich sehr intensive Färbungen bei guten Echtheitseigenschaften erreichen. Bisweilen kann es allerdings bei empfindlichen Personen zu Unverträglichkeiten gegen bestimmte Oxidationsfarbstoff-Vorstufen kommen. Ein weiterer Nachteil des permanenten Färbesystems liegt darin, daß vor allem bei häufiger Anwendung eine deutliche Verschlechterung des Haarzustandes eintritt, was unter anderem auch durch die verwendeten Oxidationsmittel und/oder der Kombination aus Ammoniak und Peroxiden zurückzuführen ist. Steht neben der Farbgebung noch der Wunsch nach milden Färbebedingungen im Vordergrund, so findet man im Markt neben Nischenprodukten, die zum Teil nur auf bestimmte Haartypen anwendbar sind und unter Umständen häufiger angewendet werden müssen, vor allem die Tönungen, die auf direktziehenden Farbstoffen aufgebaut sind.

Das Farbergebnis, das durch Tönungen erzielt werden kann, wird im Laufe der Zeit graduell schwächer, da die Farbstoffe vor allem an der Haaroberfläche adsorbiert sind und durch Shampoonieren der Haare ausgewaschen werden. Darüber hinaus können wegen physiologischer Bedenken verschiedene direktziehende Farbstoffe, wenn überhaupt, nur in eingeschränktem Maße eingesetzt werden.

Da es mit konventionellen Färbemethoden in der Regel nicht möglich ist, unter milden Färbebedingungen sehr dauerhafte Färbungen im Naturtonbereich sowie in modischen Rottönen zu erzielen, bestand weiterhin ein großer Bedarf nach einem Färbesystem, welches eine haarschonende und gleichzeitig dauerhafte Färbung ermöglicht.

Aufgabe der vorliegenden Erfindung ist es daher, ohne die Nachteile der etablierten Färbeverfahren Fasern, insbesondere Keratinfasern, wie zum Beispiel menschliche Haare, auf eine schonende und dauerhafte Weise zu färben, wobei das Färbemittel hinsichtlich seiner Farbintensität mit oxidativen Färbemitteln vergleichbar sein soll. Hierbei soll das Färbemittel auch ohne die Verwendung von Ammoniak/Peroxid- bzw. Ethanolamin/Peroxid-Kombinationen eingesetzt werden können und frei von Substanzen mit einem erhöhten Sensibilisierungspotential sein.

Es wurde nun gefunden, daß 1,3-Indandione enthaltende Färbemittel die gestellten Anforderungen in hervorragender Weise erfüllen und auch ohne den Zusatz von Oxidationsmitteln bereits bei hautneutralen pH-Werten Färbungen mit hervorragenden Echtheitseigenschaften und einer hohen Farbbrillanz ermöglichen.

Gegenstand der Erfindung sind daher Mittel zum Färben von Fasern, insbesondere Keratinfasern, wie zum Beispiel menschlichen Haaren, welche dadurch gekennzeichnet sind, daß sie mindestens ein 1,3-Indandion der allgemeinen Formel (I) enthalten 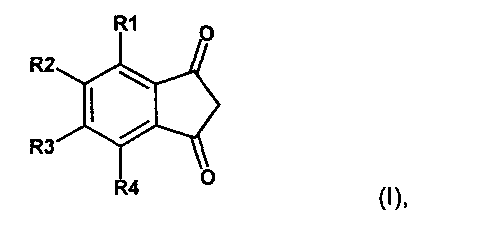 wobei die Reste R₁, R₂, R₃ und R₄ untereinander gleich oder verschieden sein können und die folgende Bedeutung haben:
Wasserstoff, ein Halogenatom, eine Nitrogruppe, eine Aminogruppe, eine Acetylaminogruppe, eine Hydroxygruppe, eine Acetoxygruppe, eine Methylgruppe oder Ethylgruppe, eine geradkettige oder verzweigte Alkoxygruppe mit 1 bis 9 Kohlenstoffatomen, eine Benzyloxygruppe, die gegebenenfalls durch eine Methylgruppe, eine Methoxygruppe oder ein Halogenatom substituiert sein kann, ein über eine Etherbrücke verknüpfter C2- bis C8-Mono-Olefinrest oder Di-Olefinrest, ein 1,3-Indandion-5-yl-Rest, eine Phenylgruppe, eine Carbonsäuregruppe, eine Carbamidgruppe, eine C1- bis C7-Carbonsäureestergruppe oder eine Phenoxypropoxygruppe; R1 und R2 beziehungsweise R2 und R3 gemeinsam eine C5- bis C6-Alkylengruppe, eine 1,3-Dioxolbrücke, eine 1,4-Dioxinbrücke, ein gegebenenfalls mit einem Halogenatom, einer Hydroxygruppe, einer Methoxygruppe oder einer Ethoxygruppe substituiertes ankondensiertes benzoaromatisches System oder ein ankondensiertes naphthoaromatisches System bilden; oder R2 und R3 gemeinsam eine 1,3-Dioxo-propan-1,3-diyl-Gruppe bilden.

Bevorzugte Verbindungen gemäß der allgemeinen Formel (I) sind Verbindungen, in denen die Reste R₁, R₂, R₃ und R₄ unabhängig voneinander die folgende Bedeutung haben:
Wasserstoff, ein Halogenatom, eine Nitrogruppe, eine Aminogruppe, eine Hydroxygruppe, eine Methylgruppe, eine geradkettige oder verzweigte Alkoxygruppe mit 1 bis 3 Kohlenstoffatomen, eine Benzyloxygruppe, die gegebenenfalls durch eine Methylgruppe oder eine Methoxygruppe substituiert sein kann oder eine Carbamidgruppe; R1 und R2 beziehungsweise R2 und R3 gemeinsam eine 1,3-Dioxolbrücke oder eine 1,4-Dioxinbrücke bilden.

Besonders bevorzugte Verbindungen sind das 1,3-Indandion, das 4-Nitro-1,3-indandion, das 5-Nitro-1,3-indandion, das 5,6-Dichlor-1,3-indandion und das 4,5,6,7-Tetrachlor-1,3-indandion.

Die erfindungsgemäßen Mittel färben Fasern, insbesondere Haare, in mittelblonden bis rotbraunen sowie rotvioletten Farbtönen.

Die 1,3-Indandione gemäß der Formel (I) sind nach aus der Literatur bekannten Herstellungsverfahren herstellbar; zudem ist ein Teil der Verbindungen der Formel (I) im Handel erhältlich. Hierbei sei insbesondere auf die Herstellung der 1,3-Indandione aus den entsprechenden Phthalsäureanhydriden gemäß der Literatur Wanag und Oschkaja, Zhur. obsc. Chim. 28, 1520 [1958] (englische Ausgabe: Journal of General Chemistry of the U.S.S.R, 28, 1570 [1958]) und der dort genannten weiterführenden Literatur verwiesen.

Die Verbindungen der Formel (I) sind zwar grundsätzlich sowohl zum Färben von natürlichen Fasern, wie zum Beispiel keratinischen Fasern wie Wolle, Baumwolle, Jute, Sisal, Leinen, Seide und Haaren, als auch von modifizierten Naturfasern, wie zum Beispiel Regeneratcellulose, Nitrocellulose, Alkylcellulose oder Hydroxyalkylcellulose oder Acetylcellulose, und synthetische Fasern, wie zum Beispiel Polyamidfasern, Polyurethanfasern oder Polyesterfasern geeignet; besonders bevorzugt ist jedoch die Verwendung der Verbindungen der Formel (I) zur Färbung von Keratinfasern und insbesondere menschlichen Haaren.

Der Gesamtgehalt an Verbindungen der Formel (I) beträgt in dem erfindungsgemäßen Färbemittel etwa 0,01 bis 5 Gewichtsprozent, vorzugsweise 0,02 bis 3 Gewichtsprozent.

Das erfindungsgemäße Mittel kann sowohl ohne Oxidationsmittel als auch unter Zugabe eines Oxidationsmittels angewandt werden, wobei im letzteren Fall der Zusatz des Oxidationsmittels in erster Linie der Erzielung von Nuancen, die heller als die zu färbende Faser sind, ("Aufhellung") dient.

Die Zugabe von Ammoniak ist insbesondere dann geboten, wenn Färbungen im hellen bis mittelblonden Bereich erzielt werden sollen. Ohne Ammoniakzugabe liegt das Farbergebnis bei modischen Rottönen.

Die Zubereitungsform des erfindungsgemäßen Färbemittels auf der Basis von Verbindungen der allgemeinen Formel (I) kann beispielsweise eine Lösung, insbesondere eine wäßrig-alkoholische Lösung, eine Creme, ein Sei oder eine Emulsion sein. Ebenfalls ist es möglich, diese Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder Aerosolschaum aus einem Druckbehälter zu entnehmen. Es ist jedoch auch möglich, daß das Mittel in Form eines Pulvers vorliegt, welches vor der Anwendung mit Wasser oder einer geeigneten Kosmetikgrundlage vermischt wird. Ebenfalls ist es möglich, daß das 1,3-Indandion in mikroverkapselter Form vorliegt.

Der pH-Wert dieses Färbemittels liegt je nach Applikationsform im Bereich von 3 bis 12, insbesondere bei einem pH-Wert von 3 bis 9, wobei die Einstellung des gewünschten alkalischen pH-Wertes vorzugsweise mit Ammoniak erfolgt, jedoch auch mit organischen Aminen wie beispielsweise Monoethanolamin oder Triethanolamin vorgenommen werden kann, während für die Einstellung eines sauren pH-Wertes je nach gewünschten pH-Wert verdünnte anorganische oder organische Säuren, wie zum Beispiel Phosphorsäure, Ascorbinsäure, Zitronensäure, Glycolsäure oder Milchsäure verwendet werden.

Selbstverständlich kann das vorstehend beschriebene Färbemittel gegebenenfalls weitere für derartige Mittel und insbesondere Haarfärbemittel übliche Zusätze, wie zum Beispiel Pflegestoffe, Netzmittel, Verdicker, Weichmacher, Konservierungsstoffe und Parfümöle sowie auch andere, in Lösungen, Cremes, Emulsionen oder Seien üblicherweise verwendete Zusätze, zum Beispiel Lösungsmittel wie Wasser, niedere aliphatische einwertige oder mehrwertige Alkohole, deren Ester und Ether, beispielsweise Alkanole, insbesondere mit 1 bis 4 C-Atomen, beispielsweise Ethanol, Propanol oder Isopropanol, Butanol, Isobutanol, zweiwertige und dreiwertige Alkohole, insbesondere solche mit 2 bis 6 C-Atomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylenglycol, Tripropylenglycol, Polypropylenglycol, niedere Alkylether von mehrwertigen Alkoholen, wie Ethylenglycolmonomethyl-ether oder Ethylenglycolmonoethylether oder Ethylenglycolmonopropyl-ether oder Ethylenglycolmonobutylether, Diethylenglycolmonomethylether oder Diethylenglycolmonoethylether, Triethylenglycolmonomethylether oder Triethylenglycolmonoethylether, Ether, wie zum Beispiel Dibutylether, Tetrahydrofuran, Dioxan, Diisopropylether, Ester wie zum Beispiel Ethylformiat, Methylformiat, Methylacetat, Ethylacetat, Propylenacetat, Butylacetat, Phenylacetat, Ethylenglycolmonoethyletheracetat und Essigsäurehydroxyethylester, Amide wie zum Beispiel Dimethylacetamid, N-Methylpyrrolidon, sowie Harnstoff, Tetramethylharnstoff und Thiodiglycol, enthalten.

Weiterhin können in dem erfindungsgemäßen Färbemittel Netzmittel oder Emulgatoren aus den Klassen der anionischen, kationischen, amphoteren, nichtionogenen oder zwitterionischen oberflächenaktiven Substanzen wie Fettalkoholsulfate, Alkansulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol; flüssige Ölkomponenten synthetischen oder natürlichen Ursprungs wie Paraffinöle, Silikonöle, Nerzöl, Olivenöl, Sonnenblumenöl, Mandelöl, Jojobaöl, Glycerin-tricaprylat; außerdem Pflegestoffe wie Lanolinderivate, Cholesterin, Pantothensäure, wasserlösliche kationische Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein. Neben Wasser kann auch ein wasserlösliches organisches Lösungsmittel oder ein Gemisch derartiger Lösungsmittel sowie ein Wasser/Lösungsmittel-Gemisch verwendet werden. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent.

Das vorstehend beschriebene Färbemittel ohne Oxidationsmittelzugabe kann weiterhin für kosmetische Mittel übliche natürliche oder synthetische Polymere beziehungsweise modifizierte Polymere natürlichen Ursprungs enthalten, wodurch gleichzeitig mit der Färbung eine Festigung der Haare erreicht wird. Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern der Polyacrylsäure, Polymethylacrylsäure und Aminoalkoholen sowie deren Salze oder Quaternisierungsprodukte, Potyacrylnitril, Polyvinylacetate sowie Copolymerisate aus derartigen Verbindungen, wie zum Beispiel Polyvinylpyrrolidon-Vinylacetat, genannt; während als natürliche Polymere oder modifizierte natürliche Polymere beispielsweise Chitosan (deacetyliertes Chitin) oder Chitosanderivate, eingesetzt werden können. Die vorgenannten Polymere können in dem erfindungsgemäßen Mittel in den für solche Mittel üblichen Mengen, insbesondere in einer Menge von etwa 1 bis 5 Gewichtsprozent, enthalten. Der pH-Wert diese Mittels mit zusätzlicher Festigung beträgt vorzugsweise etwa 3,0 bis 9,0.

Wird das erfindungsgemäße Färbemittel in Kombination mit einem Oxidationsmittel verwendet, so werden als Oxidationsmittel vorzugsweise Wasserstoffperoxid oder dessen Additionsverbindungen eingesetzt, wobei im Falle einer 3- bis 12prozentigen wäßrigen Wasserstoffperoxidlösung das Färbemittel mit dem Oxidationsmittel vorzugsweise in einem Gewichtsverhältnis von 1:1 bis 1:6 vermischt wird.

Die erfindungsgemäßen Färbemittel werden in üblicher Weise angewendet, indem man das Färbemittel vor der Behandlung erforderlichenfalls mit einem Oxidationsmittel vermischt und eine zur Färbung der Farbe ausreichende Menge der Mischung, im allgemeinen etwa 50 bis 150 Gramm, auf die Faser aufträgt. Nach einer für die Färbung ausreichenden Einwirkungszeit, die üblicherweise etwa 10 bis 30 Minuten beträgt, wird die Faser mit Wasser gespült, gegebenenfalls mit einem Shampoo gewaschen und/oder mit einer wäßrigen Lösung einer schwachen organischen Säure, wie zum Beispiel Zitronensäure oder Weinsäure, nachgespült, und sodann getrocknet. Im Falle des Färbemittels mit zusätzlicher Festigung erfolgt die Anwendung jedoch in bekannter und üblicher Weise durch Befeuchten der Faser mit dem erfindungsgemäßen Mittel, Festlegen (Einlegen) der Faser zur Frisur und anschließende Trocknung.

Hinsichtlich der färberischen Möglichkeiten bietet das erfindungsgemäße Färbemittel je nach Art und Zusammensetzung eine breite Palette verschiedener Farbnuancen, die sich von natürlichen Farbtönen bis hin zu hochmodischen, leuchtenden Nuancen erstreckt.

Die nachstehenden Beispiele sollen den Erfindungsgegenstand näher erläutern, ohne ihn auf die angeführten Beispiele zu beschränken.

### Beispiele

### Beispiele 1-6: Haarfärbelösung (basisch)

Es wird folgende Färbelösung hergestellt:
- X g: 1,3-Indandion gemäß Tabelle 1
- 10,0 g: Isopropanol
- 10,0 g: Natrium-Laurylalkoholdiglykolethersulfat (28-prozentige wäßrige Lösung)
- 10,0 g: Ammoniak 25-prozentige wäßrige Lösung
- ad 100,0 g: Wasser vollentsalzt

Das Färbemittel wird auf gebleichtes Haar aufgetragen. Nach einer Einwirkungszeit von 30 Minuten bei 40 °C werden die Haare mit Wasser gespült, shampooniert und getrocknet.

Das erhaltene Färbeergebnis ist in Tabelle 1 zusammengefaßt.

**Tabelle 1**

| **Beispiel** | **1** | **2** | **3** | **4** | **5** | **6** |
|---|---|---|---|---|---|---|
| **Einsatzmenge 1,3-Indandion** | 0,025 g | 0,05 g | 0,075 g | 0,1 g | 0,15 g | 0,2 g |
| **Farbergebnis** | hellblond | ---------> | ---------> | ---------> | ----------> | mittelblond |

Das Farbergebnis ändert sich in den Beispielen 1 bis 6 mit zunehmender Konzentration graduell von hellblond nach mittelblond.

### Beispiel 7: Haarfärbemittel in Cremeform

- 0,20 g: 1,3-Indandion
- 15,00 g: Cetylalkohol
- 3,50 g: Natrium-Laurylalkoholdiglykolethersulfat (28prozentige wäßrige Lösung)
- 3,00 g: Ammoniak (25prozentige wäßrige Lösung)
- ad 100,00 g: Wasser, entmineralisiert

Das Färbemittel wird auf gebleichtes Haar aufgetragen. Nach einer Einwirkungszeit von 30 Minuten bei 40 °C werden die Haare mit Wasser gespült, shampooniert und getrocknet.

Das Haar hat eine cognacfarbene Färbung erhalten.

### Beispiel 8: Haarfärbemittel in Gelform

- 0,20 g: 1,3-Indandion
- 7,00 g: Isopropanol
- 3,00 g: Glycerin
- 15,00 g: Ölsäure
- 10,00 g: Ammoniak (25prozentige wäßrige Lösung)
- ad 100,00 g: Wasser, entmineralisiert

Das Färbemittel mit einem pH-Wert von etwa 10 wird auf gebleichtes Tierhaar aufgetragen. Nach einer Einwirkungszeit von 30 Minuten bei 40°C werden die Haare mit Wasser gespült, shampooniert und getrocknet.

Das Haar hat eine rehbraune Färbung erhalten.

### Beispiele 9-14: Haarfärbelösung mit Verdicker

Es wird folgende Lösung hergestellt:
- X g: 1,3-Indandion gemäß Tabelle 2
- 10,0 g: Isopropanol
- 1,0 g: Hydroxyethylcellulose
- ad 100,0 g: Wasser, demineralisiert

Der pH-Wert der Haarfärbemittel in den Beispielen der Tabelle 2 beträgt 6.

Das Färbemittel wird auf gebleichtes Haar aufgetragen. Nach einer Einwirkungszeit von 30 Minuten bei 40°C werden die Haare mit Wasser gespült, shampooniert und getrocknet.

Die erhaltenen Färbungen liegen im Rotblondbereich und sind nachfolgend zusammengefaßt.

**Tabelle 2**

| **Beispiel** | **9** | **10** | **11** | **12** | **13** | **14** |
|---|---|---|---|---|---|---|
| **Einsatzmenge 1,3-Indandion** | 0,025g | 0,05g | 0,075g | 0,1g | 0,15g | 0,2g |
| **Farbergebnis** | hell-rotblond | -----------> | ----------> | ---------> | -----------> | mittel-rotblond |

Das Farbergebnis ändert sich in den Beispielen 9 bis 14 mit zunehmender Konzentration graduell von hell-rotblond nach mittel-rotblond.

### Beispiel 15: Haarfärbecreme (sauer)

- 12,0 g: Cetylstearylalcohol
- 1,4 g: Stearylalkohol mit 20 Mol Ethylenoxid (Steareth-20)
- 20,0 g: Isopropanol
- 2,0 g: 1,3-Indandion
- ad 100,00 g: Wasser, entmineralisiert

Der pH-Wert der Creme wird mit Zitronensäure auf 5,3 eingestellt.

Das Färbemittel wird auf gebleichtes Haar aufgetragen. Nach einer Einwirkungszeit von 30 Minuten bei 40°C werden die Haare mit Wasser gespült, shampooniert und getrocknet.

Das Haar hat eine sehr intensive rotviolette Färbung erhalten.

### Beispiele 16-25: Haarfärbecreme (sauer)

- 12,0 g: Cetylstearylalcohol
- 1,4 g: Stearylalkohol mit 20 Mol Ethylenoxid (Steareth-20)
- 20,0 g: Isopropanol
- 3,0 g: 1,3-Indandion
- ad 100,00 g: Wasser, entmineralisiert

Der pH-Wert der Creme wird mit Zitronensäure auf 3,3 eingestellt.

Das Färbemittel wird auf die nachfolgend angeführten Haartypen sowie Textilmaterialien aufgetragen. Nach einer Einwirkungszeit von 30 Minuten bei 40°C wird mit Wasser gespült, shampooniert und getrocknet. Die Ergebnisse sind in den Tabellen 3 und 4 zusammengefaßt.

**Tabelle 3**

| **Haarstränen** | | | |
|---|---|---|---|
| **Beispiel** | **Haartyp** | **Farbe** | **Intensität** |
| **16** | gebleichtes Tierhaar | violett | sehr stark |
| **17** | Menschenhaare mit 90% Grauanteil | weinrot | sehr stark |
| **18** | mittelblondes Menschenhaar | beaujolais dunkel | sehr stark |

**Tabelle 4**

| **Textilmaterialien** | | | |
|---|---|---|---|
| **Beispiel** | **Fasertyp** | **Farbe** | **Intensität** |
| **19** | Celluloseacetat | violett | mittel |
| **20** | Baumwolle gebleicht | rosa | mittel |
| **21** | Nylon 66 | violett | stark |
| **22** | Seide | violett | mittel |
| **23** | Polypropylen | rosa | mittel |
| **24** | Viskose | rosa | mittel |
| **25** | Wolle | weinrot | sehr stark |

### Beispiel 26: Haarfärbecreme (sauer)

- 12,0 g: Cetearylalcohol
- 1,4 g: Brij 78
- 20,0 g: Ethanol
- 1,0 g: 5-Methoxy-1,3-indandion
- ad 100,0 g: Wasser, entmineralisiert

Der pH-Wert der Creme wird mit Zitronensäure 10% in Wasser auf 5,3 eingestellt.

Das Färbemittel wird auf gebleichtes Tierhaar aufgetragen. Nach einer Einwirkungszeit von 30 Minuten bei 40°C wird mit Wasser gespült, shampooniert und getrocknet. Die Färbung ist aubergine.

### Beispiel 27: Haarfärbecreme (sauer)

- 12,0 g: Cetearylalkohol
- 1,4 g: Brij 78
- 20,0 g: Ethanol
- 2,0 g: 4-Nitro-1,3-indandion
- ad 100,00 g: Wasser, entmineralisiert

Der pH-Wert der Creme wird mit Zitronensäure 10% in Wasser auf 5,3 eingestellt.

Das Färbemittel wird auf gebleichtes Tierhaar aufgetragen. Nach einer Einwirkungszeit von 30 Minuten bei 40°C wird mit Wasser gespült, shampooniert und getrocknet. Die Färbung ist schwarz mit violettem Reflex.

### Beispiel 28: Haarfärbecreme (sauer)

- 12,0 g: Cetearylalkohol
- 1,4 g: Brij 78
- 20,0 g: Ethanol
- 1,0 g: 5,6-Dichlor-1,3-indandion
- ad 100,0 g: Wasser, entmineralisiert

Der pH-Wert der Creme wird mit Zitronensäure 10% in Wasser auf 5,3 eingestellt.

Das Färbemittel wird auf gebleichtes Tierhaar aufgetragen. Nach einer Einwirkungszeit von 30 Minuten bei 40°C wird mit Wasser gespült, shampooniert und getrocknet. Die Färbung ist aubergine hell.

### Beispiel 29: Haarfärbecreme (basisch)

- 12,0 g: Cetearylalkohol
- 1,4 g: Brij 78
- 20,0 g: Ethanol
- 1,0 g: 4,5,6,7-Tetrachlor-1,3-indandion
- ad 100,00 g: Wasser, entmineralisiert

Der pH-Wert der Creme wird mit 2n Natronlauge auf 8,0 eingestellt.

Das Färbemittel wird auf gebleichtes Tierhaar aufgetragen. Nach einer Einwirkungszeit von 30 Minuten bei 40°C wird mit Wasser gespült, shampooniert und getrocknet. Die Färbung ist lichtblond.

### Beispiel 30: Haarfärbecreme (basisch)

- 12,0 g: Cetearylalcohol
- 1,4 g: Brij 78
- 20,0 g: Ethanol
- 1,0 g: 4,5,6,7-Tetrachlor-1,3-indandion
- ad 100,00 g: Wasser, entmineralisiert

Der pH-Wert der Creme wird mit 2n Natronlauge auf 10,0 eingestellt.

Das Färbemittel wird auf gebleichtes Tierhaar aufgetragen. Nach einer Einwirkungszeit von 30 Minuten bei 40°C wird mit Wasser gespült, shampooniert und getrocknet. Die Färbung ist mittelblond.

Alle Prozentangaben stellen, soweit nicht anders angegeben, Gewichtsprozente dar.

## Patentansprüche

1. Mittel zum Färben von Fasern, dadurch gekennzeichnet, daß es mindestens ein 1,3-Indandion der allgemeinen Formel (I) enthält, 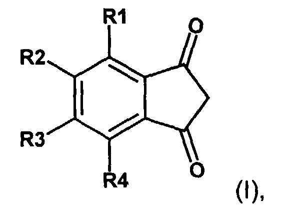 wobei die Reste R₁, R₂, R₃ und R₄ untereinander gleich oder verschieden sein können und die folgende Bedeutung haben:
Wasserstoff, ein Halogenatom, eine Nitrogruppe, eine Aminogruppe, eine Acetylaminogruppe, eine Hydroxygruppe, eine Acetoxygruppe, eine Methylgruppe oder Ethylgruppe, eine geradkettige oder verzweigte Alkoxygruppe mit 1 bis 9 Kohlenstoffatomen, eine Benzyloxygruppe, die gegebenenfalls durch eine Methylgruppe, eine Methoxygruppe oder ein Halogenatom substituiert sein kann, ein über eine Etherbrücke verknüpfter C2- bis C8-Mono-Olefinrest oder Di-Olefinrest, ein 1,3-Indandion-5-yl-Rest, eine Phenylgruppe, eine Garbonsäuregruppe, eine Carbamidgruppe, eine C1- bis C7-Carbonsäureestergruppe oder eine Phenoxypropoxygruppe; R1 und R2 beziehungsweise R2 und R3 gemeinsam eine C5- bis C6-Alkylengruppe, eine 1,3-Dioxolbrücke, eine 1,4-Dioxinbrücke, ein gegebenenfalls mit einem Halogenatom, einer Hydroxygruppe, einer Methoxygruppe oder einer Ethoxygruppe substituiertes ankondensiertes benzoaromatisches System oder ein ankondensiertes naphthoaromatisches System bilden; oder R2 und R3 eine 1,3-Dioxo-propan-1,3-diyl-Gruppe bilden.

2. Mittel nach Anspruch 1, dadurch gekennzeichnet, daß in Formel (I) die Reste R₁, R₂, R₃ und R₄ unabhängig voneinander die folgende Bedeutung haben: Wasserstoff, ein Halogenatom, eine Nitrogruppe, eine Aminogruppe, eine Hydroxygruppe, eine Methylgruppe, eine geradkettige oder verzweigte Alkoxygruppe mit 1 bis 3 Kohlenstoffatomen, eine Benzyloxygruppe, die gegebenenfalls durch eine Methylgruppe oder eine Methoxygruppe substituiert sein kann oder eine Carbamidgruppe; R1 und R2 beziehungsweise R2 und R3 gemeinsam eine 1,3-Dioxolbrücke oder eine 1,4-Dioxinbrücke bilden.

3. Mittel nach Anspruch 1 oder 2, dadurch gekennzeichnet, daß die Verbindung der Formel (I) ausgewählt ist aus 1,3-Indandion, 4-Nitro-1,3-indandion, 5-Nitro-1,3-indandion, 5,6-Dichlor-1,3-indandion und s 4,5,6,7-Tetrachlor-1,3-indandion.

4. Mittel nach einem der Ansprüche 1 bis 3, dadurch gekennzeichnet, daß es die Verbindung der Formel (I) in einer Menge von 0,01 bis 5 Gewichtsprozent enthält.

5. Mittel nach einem der Ansprüche 1 bis 4, dadurch gekennzeichnet, daß es einen pH-Wert von 3 bis 12 aufweist.

6. Mittel nach einem der Ansprüche 1 bis 5, dadurch gekennzeichnet, daß es frei von Oxidationsmitteln ist und zusätzlich ein natürliches Polymer, ein synthetisches Polymer oder ein modifiziertes Polymer natürlichen Ursprungs enthält.

7. Verfahren zum Färben von Fasern, dadurch gekennzeichnet, daß man eine zur Färbung der Faser ausreichende Menge eines Färbemittels nach einem der Ansprüche 1 bis 5 auf die Faser aufträgt, nach einer Einwirkungszeit von 10 bis 30 Minuten die Faser mit Wasser spült, gegebenenfalls mit einem Shampoo wäscht und/oder mit einer wäßrigen Lösung einer schwachen organischen Säure nachspült und sodann trocknet.
